(19) Europäisches Patentamt
European Patent Office
Office européen des brevets

(11) Veröffentlichungsnummer: **0 300 071**
**B1**

(12) # EUROPÄISCHE PATENTSCHRIFT

(45) Veröffentlichungstag der Patentschrift: **06.06.90**

(51) Int. Cl.$^5$: **C07B 63/00**, C12N 9/00

(21) Anmeldenummer: **87110575.5**

(22) Anmeldetag: **22.07.87**

(54) **Verfahren zur Abtrennung und Reinigung organischer Substanzen von Begleitstoffen.**

(43) Veröffentlichungstag der Anmeldung:
**25.01.89 Patentblatt 89/4**

(45) Bekanntmachung des Hinweises auf die Patenterteilung:
**06.06.90 Patentblatt 90/23**

(84) Benannte Vertragsstaaten:
**AT BE CH DE ES FR GB IT LI NL SE**

(56) Entgegenhaltungen:
**DE-A- 2 639 129**
**US-A- 3 579 539**

**JOURNAL OF CHROMATOGRAPHY, Band 388, 1987, Seiten 295-305, Elsevier Science Publishers B.V., Amsterdam, NL; G. JOHANSSON et al.: "Effects of organic solvents on the partitioning of enzymes in aqueous two-phase systems"**

(73) Patentinhaber: **BAYER AG,**
**D-5090 Leverkusen 1 Bayerwerk(DE)**

(72) Erfinder: **Hampe, Manfred Josef, Dr., Himbeerenweg 5,**
**D-8011 Kirchheim(DE)**
Erfinder: **Heusch, Rudolf, Dr., Paul-Klee-Strasse 85,**
**D-5090 Leverkusen 1(DE)**
Erfinder: **Heckenbach, Manfred, Dr., Kirschenweg 6,**
**D-5024 Pulheim(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung bezieht sich auf ein Verfahren zur Abtrennung und Reinigung organischer Substanzen von Begleitstoffen mittels Flüssig/Flüssig-Extraktion, wobei die eine zur Extraktion benötigte Phase zum überwiegenden Massenanteil aus einem niedermolekularen Polyalkohol und die andere Phase zum überwiegenden Massenanteil aus einer in der erstgenannten Phase nicht oder nur teilweise löslichen niedermolekularen Flüssigkeit besteht.

Ein Verfahren dieser Art ist aus US-A 3 579 539 bekannt. Gegenstand dieses Patentes ist die Abtrennung einer speziellen organischen Komponente von 4-Hydroxybenzothiophen mittels Flüssig/Flüssig-Extraktion. Die hierzu erforderlichen, flüssigen Phasen bestehen einerseits aus einem niedermolekularen Polyalkohol wie Propylenglykol und andererseits aus einer in der erstgenannten Phase nicht löslichen niedermolekularen Flüssigkeit.

Ferner ist aus der DE-PS 2 639 129 ein Verfahren bekannt, das unter Zuhilfenahme von Polymeren, wie Polyethylenglykol und Dextran oder von Polyethylenglykol und einem Salz, etwa eines Phosphates, Wasser in zwei ineinander nicht lösliche, wäßrige Phasen spaltet. Enzyme verteilen sich entsprechend ihrer Löslichkeit auf die beiden wäßrigen Phasen.

Die Trennung der Enzyme gelingt beispielsweise in Extraktionsmaschinen. Die weitere Aufarbeitung der extrahierten Enzyme umfaßt die Abtrennung der Enzyme von den Polymeren und/oder Salzen mit Hilfe von Dialyse und/oder Ultrafiltration.

Es zeigt sich jedoch, daß die Durchführung des Verfahrens in Extraktionsapparaten, in denen sich die Phasen aufgrund des Auftriebs bewegen, wegen der extrem geringen Dichteunterschiede zwischen den beiden wäßrigen Phasen und wegen der sich einstellenden sehr geringen Grenzflächenspannung problematisch ist. Beispielsweise sind nur geringe flächenbezogene Durchsätze erreichbar.

Extraktionsmaschinen hingegen bedürfen eines hohen Energieeinsatzes und sind wartungsintensiver als Extraktionsapparate. Zudem ist es mit dem bekannten Verfahren nicht möglich, die Extraktion wegen der Bildung von Eis wesentlich unter einer Temperatur von 0°C zu betreiben, wobei vor allem bei empfindlichen Enzymen mit irreversiblem Aktivitätsverlust zu rechnen ist. Weiterhin ist es technisch nicht einfach, Enzyme von extraktionssystemeigenen Polymeren, wie Polyethylenglykol und Dextran, zu trennen.

Aufgabe der vorliegenden Erfindung ist es, ein Extraktionsverfahren bereitzustellen, mit dem sich die Abtrennung und Reinigung organischer Substanzen von unerwünschten Begleitstoffen mit hohen flächenbezogenen Durchsätzen in Extraktionsapparaten, gegebenenfalls bei erheblich unter 0°C liegender Temperatur, durchführen lassen, und das weiterhin gestattet, die getrennten und/oder gereinigten organischen Substanzen auf einfache Weise von den Bestandteilen der Trägerphasen zu isolieren.

Die erfindungsgemäße Lösung dieser Aufgabe ist dadurch gekennzeichnet, daß Proteine, insbesondere Enzyme von Begleitstoffen abgetrennt bzw. gereinigt werden.

Vorzugsweise besteht die eine zur Extraktion benötigte Phase aus Glycerin. Die andere in Glycerin nicht lösliche Phase besteht dagegen vorteilhaft aus Aceton. Dabei kann dem System 0 bis 19 Massen-% Wasser beigegeben werden. Bei dem System Glycerin/Aceton sind die Viskositäten der beiden Phasen und die Dichtedifferenz im Hinblick auf die Erzielung möglichst hoher Flächen bezogener Durchsätze optimal aufeinander abgestimmt.

Bei temperaturempfindlichen Proteinen kann das erfindungsgemäße Verfahren auch bei weit unter 0°C liegenden Temperaturen durchgeführt werden.

Glycerin hat eine Dichte von 1.261 kg/m³ bei 20°C. Eine in Glycerin nicht oder nur teilweise lösliche Flüssigkeit stellt beispielsweise Amylalkohol dar. Amylalkohol hat einer Dichte von 814 kg/m³ bei 20°C. Die Dichtedifferenz zwischen der Amylalkohol- und der Glycerinphase ist hinreichend hoch, um beide Phasen in herkömmlichen Extraktionsapparaten, wie Mischer-Abscheidern, Siebbodenkolonnen, pulsierten Siebbodenkolonnen, Kolonnen mit rotierenden oder bewegten Einbauten oder Kolonnen mit geordneten Packungen oder Füllkörpern, zu kontaktieren. Die zu trennenden und/oder von Begleitstoffen zu reinigenden organischen Substanzen, insbesondere Proteine und Enzyme, können gelöst in einer der beiden Phasen an geeigneter Stelle des Extraktors eingespeist werden. Auch die Aufgabe von Aceton-Trockenextrakten oder von wäßrigen Lösungen ist möglich. Nach der Extraktion und erfolgter Phasentrennung können die getrennten und/oder gereinigten Wertstoffe aus der Amylalkoholphase durch schonende Destillation, gegebenenfalls unter Vakuum, aus der Glycerinphase, beispielsweise durch Dialyse oder Ultrafiltration, wiedergewonnen werden.

In weiterer Ausgestaltung der Erfindung wird vorgeschlagen, als in Glycerin nicht oder nur teilweise lösliche Flüssigkeit Aceton einzusetzen. Aceton und Glycerin weisen eine teilweise gegenseitige Löslichkeit auf. Bei 20°C lösen sich 10,4 % Aceton in Glycerin und 6,4 % Glycerin in Aceton. Die Dichten der beiden Phasen betragen 1.210 kg/m³ bzw. 810 kg/m³, die Viskositäten 350 mPas bzw. 0,4 mPas, die Grenzflächenspannung beträgt etwa 6,4 $mNm^{-1}$. Auch bei Zusatz geringer Mengen Wasser bleibt die Mischungslücke zwischen Aceton und Glycerin erhalten. Beispielsweise stehen miteinander im Gleichgewicht eine Glycerinphase mit 70,3 % Glycerin und 14,3 % Wasser sowie eine Acetonphase mit 86,9 % Aceton und 4,4 % Wasser. Die Dichten der Phasen betragen 1.120 kg/m³ bzw. 830 kg/m³, die Viskositäten 30 mPas bzw. 0,45 mPas. Durch den Zusatz verschiedener Anteile an Wasser läßt sich einerseits die Viskosität der Glycerinphase, andererseits die Löslichkeit und damit die Verteilung der gelösten Substanzen beeinflussen und gemäß den Anforderungen des Kontaktapparates und/oder des Stoffsystems einstellen. In einer besonders vorteilhaften Ausführung lassen sich die Glycerin- und Acetonphase in einem Fallfilm-Kontaktapparat im Gegenstrom zueinander führen, jedoch können auch die anderen Extraktionsapparate und -maschinen prinzipiell verwendet werden. Die hohen Dichteunterschiede sowie die relativ geringen Viskositäten begünstigen eine hohe Flächenbelastung der Extraktionsapparate sowie eine leichte Abscheidbarkeit der Phasen.

Es wird weiterhin vorgeschlagen, die Extraktion nach Möglichkeit bei einer weit unter 0°C liegenden Temperatur durchzuführen. Hierdurch gelingt es, z.B. temperaturempfindliche Proteine und bevorzugterweise Enzyme ohne nennenswerte Produktschädigung zu trennen bzw. zu reinigen.

Das oben angeführte Gemisch mit 70,3 % Glycerin und 14,3 % Wasser erstarrt z.B. erst unterhalb -39°C, die korrespondierende Acetonphase bei noch weit tieferer Temperatur. Man kann daher den Anforderungen des Produktes entsprechend die Prozeßtemperatur bis zu -39°C, bei weiterem Zusatz von Wasser auch noch darunter, absenken.

Ein weiterer Vorteil der Erfindung ist die Verwendung niedermolekularer Komponenten, die insbesondere die Abtrennung von Proteinen, vorzugsweise von Enzymen, erheblich erleichtert. Z.B. lassen sich thermische Trennoperationen, wie Destillation, Vakuumdestillation, oder Membrantrennverfahren, wie Dialyse und/oder Ultrafiltration, anwenden, um die Proteine und insbesondere die Enzyme rückstandsfrei von den verwendeten Lösungsmitteln zu trennen.

Ausführungsbeispiel 1

53,28 g Wasser, 209,1 g Aceton und 132,5 g Glycerin werden in einer Stufe eines Mischer-Abscheiders zusammen mit 11,54 Einheiten Peroxidase versetzt. Die Temperatur beträgt 25°C. Nach dem Rühren und Absetzen der Phasen werden die Aceton- und die Glycerinphase getrennt und jede für sich einer mehrstündigen Dialyse an einem Membranschlauch aus regenerierter Zellulose mit einer Trenngrenze von 3.500 Dalton unterworfen. Nach der Dialyse enthält der Schlauchinhalt weder Aceton noch Glycerin, sondern lediglich Wasser und Enzym. Eine Bestimmung der Enzymaktivität zeigt, daß in der dialysierten Glycerinphase insgesamt 6,69 Einheiten, in der dialysierten Acetonphase 0,04 Einheiten Peroxidase vorhanden waren. Der Verteilungsfaktor von Peroxidase zwischen Glycerin- und Acetonphase beträgt 151:1. Durch die Extraktion und die nachfolgende Dialyse ist ein Aktivitätsverlust von insgesamt 42 % eingetreten.

Ausführungsbeispiel 2

Bei ähnlicher Zusammensetzung wie in Beispiel 1 werden 810 Einheiten alkalische Phosphatase extrahiert und der gleichen Verfahrensweise wie in Beispiel 1 unterzogen. Alkalische Phosphatase verteilt sich im Verhältnis 70:1 zwischen Glycerin- und Acetonphase. Nach der Dialyse werden insgesamt 551 Einheiten alkalische Phosphatase festgestellt. Das entspricht einer Ausbeute von 68 %.

Beispiel 3

(Verteilung eines Triarylmethan-Farbstoffes)

Zu gleichen Volumenteilen einer acetonreichen Phase mit etwa 75 mol-% Aceton, 20 mol-% Wasser und 5 mol-% Glycerin sowie einer glycerinreichen Phase mit etwa 28 mol-% Glycerin, 50 mol-% Wasser und 22 mol-% Aceton wurden unterschiedliche Mengen des blauen Triarylmethan-Farbstoffs

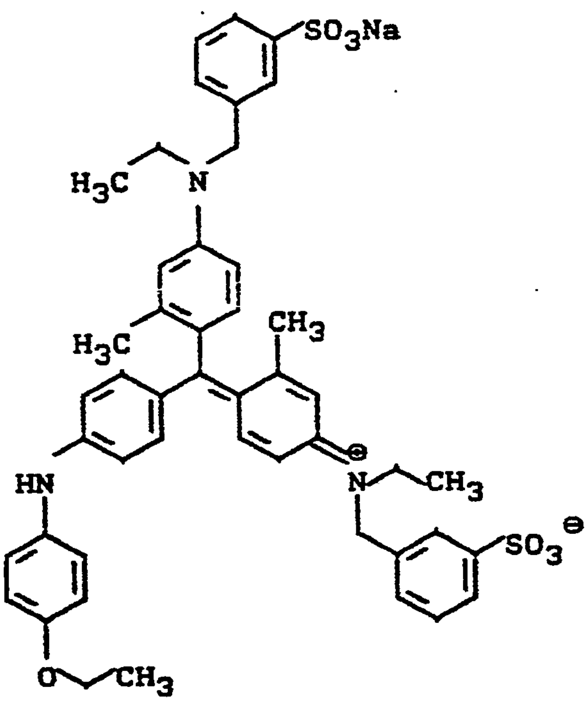

(C.I. 42655, bezogen von Fluka, Katalog-Nr. 27815) zugefügt, so daß die auf das Gesamtvolumen beider Phasen bezogene Farbstoffkonzentration jeweils 0,5, 1, 5, 10, 30, 80 und 120 g/l betrug. Der Farbstoff verteilte sich zwischen den beiden Phasen so, daß sich in der Acetonphase die niedrigere, in der Glycerinphase die höhere Farbstoffkonzentration einstellte. Die experimentell mit einer Fehlerbreite von 10 % bestimmten Verteilungskoeffizienten betrugen jeweils 0,48, 0,39, 0,45, 0,39, 0,24, 0,21 und 0,17.

Durch Zusatz von Hilfsstoffen, beispielsweise durch Zusatz von Tetradodecylammoniumbromid ließen sich die Verteilungskoeffizienten erheblich beeinflussen. Fügte man soviel Tetradodecylammoniumbromid hinzu, daß die auf das Gesamtvolumen beider Phasen bezogene Konzentration an Tetradodecylammoniumbromid jeweils 10 g/l betrug, so erhöhten sich die den obengenannten Farbstoffkonzentrationen entsprechenden Verteilungskoeffizienten zugunsten einer höheren Beladung der Acetonphase auf 8,0, 7,6, 4,2, 2,5, 0,89.

Fügte man soviel Tetradodecylammoniumbromid hinzu, daß die auf das Gesamtvolumen beider Phasen bezogene Konzentration an Tetradodecylammoniumbromid jeweils 30 g/l betrug, so erhöhten sich die den obengenannten Farbstoffkonzentrationen entsprechenden Verteilungskoeffizienten weiter auf 12,4, 14,4, 9,1, 6,6, 2,6, 0,8 und 0,5. 0,29 und 0,25.

Beispiel 4

(Verteilung eines Anthrachinon-Farbstoffs)

Zu gleichen Volumenteilen einer acetonreichen Phase mit etwa 75 mol-% Aceton, 20 Mol-% Wasser und 5 mol-% Glycerin sowie einer glycerinreichen Phase mit etwa 28 mol-% Glycerin, 50 mol-% Wasser und 22 mol-% Aceton werden unterschiedliche Mengen des blauen Anthrachinon-Farbstoffs

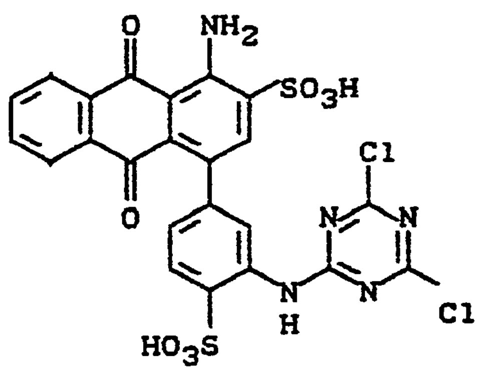

(C.I. 61205, bezogen von Fluka, Katalog-Nr. 81683) zugefügt, so daß die auf das Gesamtvolumen beider Phasen bezogene Farbstoffkonzentration jeweils 0,5, 1, 5, 10, 30, 50 und 90 g/l betrug. Der Farbstoff verteilte sich zwischen den beiden Phasen so, daß sich in der Acetonphase die niedrigere, in der Glycerinphase die höhere Farbstoffkonzentration einstellte. Die experimentell mit einer Fehlerbreite von etwa 10 % bestimmten Verteilungskoeffizienten betrugen jeweils 0,20, 0,22, 0,23, 0,23, 0,29, 0,26 und 0,16.

Durch Zusatz von Hilfsstoffen, beispielsweise durch Zusatz von Tetradodecylammoniumbromid ließen sich die Verteilungskoeffizienten erheblich beeinflussen. Fügte man soviel Tetradodecylammoniumbromid hinzu, daß die auf das Gesamtvolumen beider Phasen bezogene Konzentration an Tetradodecylammoniumbromid jeweils 10 g/l betrug, so erhöhten sich die den obengenannten Farbstoffkonzentrationen entsprechenden Verteilungskoeffizienten zugunsten einer höheren Beladung der Acetonphase auf 63,0, 69,6, 11,5, 2,8, 0,76, 0,49 und 0,32.

Fügte man soviel Tetradodecylammoniumbromid hinzu, daß die auf das Gesamtvolumen beider Phasen bezogene Konzentration an Tetradodecylammoniumbromid jeweils 30 g/l betrug, so erhöhten sich die den obengenannten Farbstoffkonzentrationen entsprechenden Verteilungskoeffizienten weiter auf 122, 178, 167, 48, 4,5, 1,9, und 0,8.

Beispiel 5

(Verteilung eines Pharmawirkstoffs)

Zu 0,5 ml einer acetonreichen Phase mit etwa 75 mol-% Aceton, 20 mol-% Wasser und 5 mol-% Glycerin sowie 0,5 ml einer glycerinreichen Phase mit etwa 28 mol-% Glycerin, 50 mol-% Wasser und 22 mol-% Aceton wurden 1 mg des Pharmawirkstoffes Oxytocin zugefügt. Das Oxytocin verteilte sich zwischen der Aceton und der Glycerinphase im Verhältnis 1:6.

Beispiel 6

22 g Wasser, 40 g Rohrzucker und 38 g Aceton werden in einem Scheidetrichter bei Raumtemperatur intensiv geschüttelt und anschließend ruhen gelassen. Es bilden sich zwei Phasen aus, wobei die obere, vorwiegend Aceton-haltige Phase 47 % des Gesamtvolumens ausmacht.

Diamantechtblau BL geht beim Schütteln in diesem System überwiegend in die untere, rohrzuckerreiche Phase. Aus der photometrischen Bestimmung der Konzentration in beiden Phasen folgt ein Verteilungskoeffizient von 36.

Beispiel 7

In dem unter Beispiel 6 beschriebenen System verteilt sich der Farbstoff Resolindunkelblau 5R überwiegend in die obere acetonreiche Phase. Der Verteilungskoeffizient beträgt hierbei 230.

Beispiel 8

24 g Wasser, 37 g Sorbit und 39 g Aceton werden in einem Scheidetrichter bei Raumtemperatur intensiv geschüttelt. Nach Ruhenlassen bilden sich zwei Phasen aus, wobei die obere, vorwiegend Aceton enthaltende Phase und die untere vorwiegend Sorbit enthaltende Phase, je 50 % des Gesamtvolumens ausmachen.

Diamantechtblau BL geht beim Schütteln in diesem System überwiegend in die untere sorbithaltige Phase. Der aus der photometrischen Bestimmung der Konzentration in beiden Phasen erhaltene Verteilungskoeffizient ist 94.

Beispiel 9

In dem unter Beispiel 8 beschriebenen System verteilt sich der Farbstoff Resolindunkelblau 5R überwiegend in die obere acetonreiche Phase. Der Verteilungskoeffizient ist 500.

Beispiel 10

13,5 g Wasser, 52,9 g Aceton und 33,5 g Glycerin werden zusammen mit 500 mg Resolindunkelblau 5R in einem Scheidetrichter 15 Minuten bei Raumtemperatur kräftig geschüttelt. Danach läßt man das Gemisch 1 bis 2 Stunden ruhig stehen. Es scheiden sich 2 Phasen ab, die sorgfältig getrennt werden.

Die obere, vorwiegend Aceton-haltige Phase mit 46,3 g enthält 456 mg Farbstoff, die spektroskopisch bei 554 nm bestimmt wurden. Nach Abdampfen und Trocknen bei 80°C im Vakuumschrank wurden 450 mg Farbstoff ausgewogen.

Die untere, vorwiegend Glycerin enthaltende Phase mit 51,7 g enthält nach der spektroskopischen Bestimmung 4,9 mg Farbstoff, die nicht isoliert wurden.

Der Verteilungskoeffizient betrug 92, die Ausbeute 88 %.

Beispiel 11

13,5 g Wasser, 52,9 g Aceton und 33,5 g Glycerin, 502,4 mg Diamantechtblau BL nach Beispiel 10:

Obere Phase: 43,9 g mit 14,2 mg Farbstoff, spektroskopisch bestimmt bei 526 nm.

Untere Phase: 54,3 g mit 460 mg Farbstoff, spektroskopisch bestimmt bei 526 nm.
Isoliert wurden 456 mg Farbstoff = 91 % Ausbeute.
Verteilungskoeffizient 32,5.

Beispiel 12

12,8 g Wasser, 55,8 g Methylethylketon und 31,6 g Glycerin, 260 mg Resolindunkelblau 5R nach Beispiel 10:

Obere Phase: 55,9 g mit 251 mg Farbstoff, spektroskopisch bestimmt bei 554 nm.
Isoliert wurden 250 mg Farbstoff = 96 % Ausbeute.
Verteilungskoeffizient 5020.

Beispiel 13

4,1 g Wasser, 15,9 g Aceton und 10,1 g Glycerin, 103,4 mg Tetracyclin nach Beispiel 10:

Obere Phase: 11,9 g mit 11,7 mg Tetracyclin, spektroskopisch bestimmt bei 357 nm.

Untere Phase: 16,5 g mit 87,4 mg Tetracyclin, spektroskopisch bestimmt bei 257 nm = 84,5 % Ausbeute.
Verteilungskoeffizient 7,5.

Beispiel 14

3,8 g Wasser, 16,7 Methylethylketon und 9,8 g Glycerin, 100,2 mg Terramycin nach Beispiel 10:

Obere Phase: 16,1 g mit 3,5 mg Terramycin, spektroskopisch bestimmt bei 357 nm.

Untere Phase: 13,4 g mit 91,5 mg Terramycin, spektroskopisch bestimmt bei 357 nm = 91 % Ausbeute.

Verteilungskoeffizient 26

Beispiel 15

60 g Wasser, 290,5 g Methylethylketon und 149 g Glycerin, 5,0 g Gammexan nach Beispiel 10:

Obere Phase: 291 g mit 4,6 g Gammexan, polarographisch bestimmt = 92 % Ausbeute.

Untere Phase: 210 g mit 0,03 g Gammexan, polarographisch bestimmt.
Verteilungskoeffizient 153.

## Patentansprüche

1. Verfahren zur Abtrennung und Reinigung organischer Substanzen von Begleitstoffen mittels Flüssig/Flüssig-Extraktion, wobei die eine zur Extraktion benötigte Phase zum überwiegenden Massenanteil aus einem niedermolekularen Polyalkohol und die andere Phase zum überwiegenden Massenanteil aus einer in der erstgenannten Phase nicht oder nur Teilweise löslichen niedermolekularen Flüssigkeit besteht, dadurch gekennzeichnet, daß Proteine, insbesondere Enzyme, von Begleitstoffen abgetrennt bwz. gereinigt werden.

2. Verfahren nach Anspruch 1, dadurch gekennzeichnet, daß die erstgenannte zur Extraktion benötigte Phase aus Glycerin besteht.

3. Verfahren nach den Ansprüchen 1 und 2, dadurch gekennzeichnet, daß die in Glycerin nicht oder nur teilweise lösliche Flüssigkeit Aceton darstellt und dem System 0 bis 19 Massenprozent Wasser beigegeben werden.

4. Verfahren nach den Ansprüchen 1 bis 3, dadurch gekennzeichnet, daß die Flüssig/Flüssig-Extraktion bei weit unter 0°C liegender Temperatur durchgeführt wird.

## Claims

1. A process for the separation and removal of impurities from organic substances by liquid-liquid extraction, one of the phases required for extraction consisting predominantly by weight of a low molecular weight polyalcohol and the other phase consisting predominantly by weight of a low molecular weight liquid insoluble or only partly soluble in the first phase, characterized in that impurities are separated off and removed from proteins, particularly enzymes.

2. A prozess as claimed in claim 1, characterized in that the first phase required for extraction consists of glycerol.

3. A process as claimed in claims 1 and 2, characterized in that the liquid insoluble or only partly soluble in glycerol is acetone and 0 to 19% by weight water is added to the system.

4. A process as claimed in claims 1 to 3, characterized in that the liquid/liquid extraction is carried out at a temperature far below 0°C.

## Revendications

1. Procédé de séparation et d'épuration de substances organiques des impuretés qui les accompagnent moyennant une extraction liquide/liquide, une des phases nécessaires pour l'extraction étant constituée dans une mesure prépondérante, d'un polyalcool de faible poids moléculaire, tandis que l'autre phase est constituée, dans une mesure prépondérante, d'un liquide de faible poids moléculaire non soluble ou uniquement partiellement soluble ou uniquement partiellement soluble dans la phase mentionnée en premier lieu, caractérisé en ce que les protéines, en particulier, les enzymes, sont séparées ou épurées des impuretés qui les accompagnent.

2. Procédé selon la revendication 1, caractérisé en ce que la phase mentionnée en premier lieu et nécessaire pour l'extraction est constituée de glycérol.

3. Procédé selon les revendications 1 et 2, caractérisé en ce que le liquide non soluble ou uniquement partiellement soluble dans le glycérol est l'acétone, tandis qu'on ajoute, au système, 0 à 19% en masse d'eau.

4. Procédé selon les revendications 1 à 3, caractérisé en ce qu'on effectue l'extraction liquide/liquide à une température nettement inférieure à 0°C.